# EUROPEAN PATENT APPLICATION

(11) **EP 2 098 245 A1**
(43) Date of publication of application: **09.09.2009**
(21) Application number: 07816485.2
(22) Date of filing: 29.09.2007
(51) Int. Cl.: A61K 38/46, A61P 25/00, A61P 29/00, A61P 11/00, A61P 1/00, A61P 37/00

(54) **USE OF CHOLINESTERASE FOR MANUFACTURING AN ANTI-TACHYKININS MEDICAMENT**

(30) Priority: 30.11.2006 CN 200610157321
(71) Applicant: Shenzhen Dingxin Bio-medicine Tech Development Ltd, Shenzhen, Guangdong 518-020 (CN); South China Agricaltural University, Guangzhou, Guangdong 510-642 (CN)
(72) Inventor: TAO, Guoliang, Shenzhen Guangdong 518-020 (CN); XU, Hanhong, Guangdong 510-642 (CN)
(74) Representative: Hryszkiewicz, Danuta
(86) International application number: PCT/CN2007/002871
(87) International publication number: WO 2008/064552

(57) **Abstract**

The present invention provides a use of cholinesterase for manufacturing a medicament for the treatment or prevention of mammalian diseases mediated by tachykinins. The diseases include pain, inflammation, disorders of central nervous system, disorders of respiratory system, gastrointestinal disorders, allergy, neuropathy, immune-related disease, rheumatism, dermatosis, and chronic fatigue syndrome. The cholinesterase may be acetylcholinesterase, butylcholinesterase, or isoenzyme or derivatives thereof. They may be obtained from animal cells, tissues, blood, from plant, or by method of gene recombination.

## Description

This invention relates to pharmaceutical compositions and their use for the treatment or prevention of diseases mediated by tachykinins, and more particularly to pharmaceutical compositions comprising cholinesterase which hydrolyzes amino acid firstly from the site of C-terminal Leu-Met for the treatment or prevention of diseases mediated by tachykinins.

Tachykinin (TK) peptides are one of the largest family of neuropeptides found in mammals including humans. They are characterized by a common C-terminal sequence, Phe-X-Gly-Leu-Met-NH₂. Tachykinins (TKs) were so named due to their ability to rapidly induce contraction of gut tissue. TK peptides, which include substance P (SP), neurokinin A (NKA), and neurokinin B (NKB), are widely present in the central and peripheral nervous systems, where they act as neurotransmitters/modulators, as well as growth factors.

Studies have shown that SP acts on various immune cells and plays a major role in neuroendocrine regulation of the immune system. SP also affects the cytokine synthesis in activated lymphocytes, and mediates and regulates immune inflammatory response. SP plays an important role in immunocyte recruitment during inflammation. SP affects the cytokine synthesis of assistant T cells. SP and its C-terminal fragments can initiate and delay neutrophil apoptosis, which means that SP plays a role in inflammation deferment. Furthermore, SP is a potent chemotactic factor for neutrophil; 10⁻⁹ mol/L of SP can directly induce neutrophil to transfer to the inflammation site. SP can activate mast cells, and cause intracellular Ca²⁺ mobilization, cell degranulation and histamine release. There are many SP-binding sites on the surface of human skin Langerhans cells; if they are activated, antigen-presenting will be inhibited. SP can also induce bone marrow cells to produce IL-1 and IL-6.

The influence of SP on various systems of the human body is summarized below:

1) Nervous system: SP functions as an excitatory medium affecting the target tissue from primary introduction to terminals release, and the process is often involved in pain transmission;

2) Digestive system: SP contracts gastrointestinal smooth muscles, stimulates more secretion of saliva, and promotes water and electrolyte secretion in large intestine and small intestine;

3) Circulatory system: SP expands blood vessels, increases vascular permeability and promotes plasma protein exudation; intraventricular injection of SP can raise the average blood pressure in rats;

4) Respiratory system: SP nerve cell participates in air-exchange startup in shortage of oxygen, strengthens the tension of gastrointestinal smooth muscles, convulses bronchia and causes pulmonary edema;

5) Urinary system: SP contracts ureter and bladder smooth muscles, increases the releasing of ADH, increases renal blood flow and urine; and

6) Integumentary system: injection of SP in human skin causes local red swelling and pruritus; when skin lesions occur, SP participates in local inflammatory response and immune response.

It is important to note that SP is not only an important neurotransmitter, but also a chemical language common among the nervous system, the endocrine system and the immune system. It is a significant messenger for communication transmissions and body's response regulation (Fang Xiubin, Neuropeptides and neurotrophic factor, 42-45, People's Health Publishing House). Due to multiple physiological functions in various tissues, SP receptor agonists and antagonists have bright application prospects.

The activity of SP is closely related to its structure. When the amino acid of the C-terminal is fragmented off, the activity is significantly decreased or eliminated. Thus, it is known that the six amino acid chain at C-terminal is indispensable for the biological activity of SP. When the 6^{th}, 7^{th}, 8^{th}, 9^{th} and 11^{th} amino acid residues of SP were substituted with corresponding D-amino acid, the receptor-binding capacity was nearly completely lost (Lu Changlin, et al., Neuropeptide: Basis and Clinic,134, Second Military Medical University Press).

SP and other TKs are related to the pathophysiology of certain diseases. For example, SP participates in signal transduction in pain or migraine (B. E. B. Sandberg, et al., Journal of Medicinal Chemistry, 25, 1009 (1982); L.Urban et al. TINS, 1994, 17, 432-438; Seguin, et al. Pain, 1995, 61, 326-343; S. H. Buck, 1994, "The Tachykinin Receptors", Humana Press, Totowa, N.J.), it relates to central nervous system diseases such as anxiety and schizophrenia (C. A. Maggi, et al., Autonomic Pharmacol., 1993, 13, 23-93; M. Otsuka and K. Yoshioka Physiol. Rev. 1993, 73, 229-308), to respiratory system diseases, such as asthma (J. Mizrahi et al., Pharmacology, 1982, 25, 39-50; C.Advenier et al., Eur. Reespir. J., 1997, 10, 1892-1906; C. Advenier and X.Emonds-Alt, Pulmonary Pharmacol., 1996, 9, 329-333), to allergy and inflammation (S. H. Buck, 1994,"The Tachykinin Receptors"), and to rheumatism, incl. muscular rheumatism, gastrointestinal disorders and gastrointestinal diseases, such as ulcerative colitis and Crohn colitis (P. Holzer and U. Holzer-Petsche, Pharmacol. Ther., 1997, 73, 173-217 and 219-263; D. Regoli, F. Sicuteri, Trends in Cluster Headache, 85-95, Elsevier Scientific Publishers, 1987).

Due to a close relationship between TKs, incl. SP, and the pathophysiology of certain diseases, SP receptor agonists and antagonists have bright application prospects. TK antagonists, such as SP antagonists, have been disclosed in literature, e.g., Chinese Pat. Nos. 02131556.6, 94194790.4, 94195008.5, 95194416.9, 96123888.7, 95195280.8, 94192362.2, 93114371.3, 93100018.1, 99810915.0, 99810017. X and U.S .Pat. Nos. US 6,521,621, 6,329,394, 6,197,772, and 5,939,433. Particularly, specific receptor antagonists of tachykinin receptor subtypes were disclosed, and all were chemosynthetic. U.S. Pat. No. 6,709,651 discloses an SP antagonist which is a peptidase and can selectively cleave peptides at the Xaa-Pro site. The mechanism lies in that the peptidase cleaves receptor-specific binding sites of SP and inhibits binding of SP.

Cholinesterase is a class of glycoproteins, including acetylcholinesterase and butyrylcholinesterase, which catalyze the hydrolysis of the neurotransmitter acetylcholine into choline and acetic acid. Acetylcholinesterase (EC 3.1.1.7) (AChE), also known as true cholinesterase, is found mainly at neuromuscular junctions and cholinergic synapses in the central nervous system, where its activity serves to terminate synaptic transmission. It is also found on the red blood cell membranes, where it constitutes the Yt blood group antigen. AChE has a very high catalytic activity and specificity.

Butyrylcholinesterase (EC 3.1.1.8) (BChE), also known as serum cholinesterase, pseudocholinesterase, or non-specific cholinesterase, is mainly found in Glial cells, plasma, liver, kidney, and small intestine. The enzyme has a low specificity to Ach, and can hydrolyze other choline esters, such as succinylcholine.

There are no prior reports of using cholinesterase in the treatment or prevention of SP-mediated diseases.

In view of the above-described problems, it is one objective of the invention to provide a pharmaceutical composition and its use for the treatment or prevention of tachykinin-mediated diseases in mammals.

To achieve the above objectives, in accordance with one embodiment of the invention, provided is a pharmaceutical composition and its use for the treatment or prevention of tachykinin-mediated diseases in mammals. The diseases are selected from pain, inflammation, central nervous system diseases, respiratory diseases, gastrointestinal disorders and gastrointestinal diseases, allergy, neuropathological diseases, diseases related to immune enhancement or suppression, rheumatism, skin diseases, and/or chronic fatigue syndrome.

Cholinesterase is used as a monomer or a mixture.

In a preferred embodiment, cholinesterase is selected from the group consisting of acetylcholinesterase, butyrylcholinesterase, acetylcholinesterase or butyrylcholinesterase isozymes and derivatives, a recombinant enzyme derivative of acetylcholinesterase or butyrylcholinesterase.

In a preferred embodiment, cholinesterase is a non-homogeneous crude extract isolated and purified from tissues of animals and plants or blood, or from an expression vector obtained by gene recombinant technology, and has cholinesterase activity when taking acetylcholine or butylcholine as substrate to detect.

In a preferred embodiment, the dose range of cholinesterase is between 0.01 µg and 1 mg.

In another embodiment of the invention, provided is a pharmaceutical composition for the treatment or prevention of tachykinin-mediated diseases in mammals, comprising a therapeutically effective amount of cholinesterase antagonising tachykinin. Cholinesterase is provided as a monomer or a mixture.

In another embodiment of the invention, provided is a formulation for the treatment or prevention of tachykinin-mediated diseases in mammals, comprising the above-mentioned pharmaceutical composition and pharmaceutically acceptable excipients.

Still in another embodiment of the invention, provided is a method of preparing a formulation for the treatment or prevention of tachykinin-mediated diseases in mammals, comprising: homogenously mixing the above-mentioned pharmaceutical composition with a pharmaceutically acceptable excipient.

Without wishing to be bound by theory, the basic principle of the invention is speculated to include cleavage of C-terminal amino acid residues of Substance P by cholinesterase or its isoenzyme at Leu-Met. Subsequently, C-terminal polypeptide chain is hydrolyzed at multiple sites. Accordingly, the content of SP which is abnormally high in a patient is decreased, and the diseases mediated by tachykinin are treated or prevented.

Firstly, cholinesterase hydrolyzes C-terminal amino acid residues of Substance P from the site of Met¹¹, then at multiple sites of C-terminal of polypeptide, to yield 1-10, 1-9, 1-7, or 1-5 peptide fragments. The fragments cannot be activated by or bound to a receptor. Herein cholinesterase plays a role of "an antagonist" by decreasing the effectiveness of the original polypeptide, so it can treat or prevent diseases systemically or locally which are treatable with SP or tachykinin-antagonists.

Specifically, in this invention, a pharmaceutical composition comprising cholinesterase or its isoenzyme firstly hydrolyzes C-terminal amino acid residues of Substance P at Leu-Met, and then at multiple sites of the C-terminal of polypeptide including tachykinin such as SP, so that tachykinin loses its biological activity and receptor-binding capacity. Herein, the pharmaceutical composition comprising cholinesterase or its isoenzyme functions as an antagonist. Tachykinin-mediated disease can be treated or prevented by decreasing tachykinin-mediated immunoregulation or nerve transduction. The terms "treat," "treating," and "treatement," as used herein, mean administering to a patient in need thereof a therapeutically effective amount of pharmaceutical composition comprising cholinesterase.

The pharmaceutical composition of the invention antagonizes tachykinins by hydrolyzing tachykinin, particularly antagonizes SP by hydrolyzing SP, so it is suitable for use in medicine, particularly for the treatment and prevention of tachykinin-mediated disease in mammals.

The applications of this invention include but are not limited to:

Analgesic, particularly for the treatment of pain caused by surgery or sports trauma; brachial plexus pain; chronic pain caused by osteoarthritis or rheumatoid arthritis; migraine; herpetic neuralgia, inter-pulse and/or peripheral neuralgia, diabetic neuralgia, neuralgia caused by chemotherapy or AIDS; phantom pain; toothache; cancer pain; dysmenorrhea, menstrual pain; pain caused by sciatic nerve or disc tenesmus; gout; skin pain related to prurigo or scars; surrounding pain such as cervical vertebral pain, shoulder and elbow pain caused by nerve, blood vessel or muscle damage.

Anti-inflammatory agent, particularly for the treatment of asthma, influenza, bronchitis (especially COPD), cough, allergic reaction, bronchospasm; rheumatoid arthritis, osteoarthritis; gastrointestinal inflammation such as Crohn's disease, segmental ileitis, ulcerative colitis, gastrointestinal disorders caused by infection; and cystitis.

Treatment of allergy, particularly of skin allergy such as rubella, contact dermatitis and rhinitis.

Treatment of central nervous system diseases, particularly of anxiety, depression, schizophrenia, manic-depressive psychosis, sexual dysfunction, dementia, stress-related disease, sleep disturbance, cognitive impairment, affective disorder, schizoaffective disorder, eating disorders, neuropathic barriers, addiction obstacles, personality disorder, doldrums, drug dependence, panic and other CNS-related diseases.

Treatment of acute or delayed nausea and vomiting caused by chemotherapy, dialysis, toxins, dizziness, high-altitude, dyspepsia and so on.

Treatment of gastrointestinal systemic diseases such as intestinal stress syndrome (IBS), diarrhea, excessive secretion, gastritis, fecal incontinence, food allergy.

Treatment of skin lesions such as psoriasis, atopic dermatitis, urticaria, pruritus, spots, and particularly sunburns.

Treatment of vascular spasm disease, fibrous tissue and collagen diseases, and diseases related to immune enhancement or suppression, rheumatism and weight control.

Treatment of high blood pressure, stroke, epilepsy, head trauma, spinal cord trauma, ischemic nerve injury such as central ischemic injury caused by stroke or vascular occlusion, excitotoxic nerve damage such as stroke and epilepsy, and muscle atrophic lateral sclerosis.

Treatment of chronic fatigue syndrome.

Cholinesterase can rapidly inactivate tachykinin, a pro-inflammatory cytokine, in tissue and body fluids, so excessive inflammation in the course of high level of immunization and intense inflammatory response in the course of compromised immune are limited. Patients with cancer, AIDS or organ transplantation often suffer from compromised immunity. Furthermore, local and systemic inflammatory response syndrome in the course of immunological recovery is also limited. Cancer patients, besides suffering from damage caused by cancer itself, more suffer from the inflammatory lesions of other parts of the body caused by cancer, side effects caused by chemotherapy and radiotherapy and inflammation caused by compromised immunity due to medication. These sufferings have a decisive effect on cancer patients' lifespan and quality of life. Therefore, it is beneficial for patients having cancer to be administered the pharmaceutical composition of the invention.

Cholinesterase can rapidly inactivate tachykinin, a significant messenger for communication transmission and response regulation, in tissue and body fluids. When the body is attacked by new exogenous antigens such as SARS virus, bird flu influenza virus, or fulminant influenza, extreme reactions may occur, which can lead to drastic change of health status and even death. Administering the pharmaceutical composition of the invention can prevent and treat extreme immunoreactions.

The pharmaceutical composition of the invention can be used to prepare cosmetics, and particularly used to prepare a reagent for inhibiting the release of SP-induced histamine from mast cells.

Due to different sources and purification means, the specific activity of cholinesterase varies greatly, ranging from several dozen units to tens of thousands of units per mg protein. The specific activity range is applicable in this invention. For example, for a dry matter with the specific activity of 1,000 units (U) per mg protein, the therapeutically effective amount of the invention is between 0.01 µg and 1 mg, particularly between 0.1µg and 1 mg.

The pharmaceutical composition of the invention is generally formulated in form of aqueous solution, freeze-dried powder, or freeze-dried powder-solvent complex.

The invention teaches a pharmaceutical composition comprising cholinesterase and its use for the treatment or prevention tachykinin-induced diseases. The treatment or prevention has proved effective and has low to no side effects. Cholinesterase of the invention is selected from acetylcholinesterase, butyrylcholinesterase, its isoenzyme or derivatives, which are isolated from animal cells, tissues or blood, plants, or obtained by gene recombination technology; the sources are extensive. Furthermore, the enzyme does not need to be highly purified. All these advantages show the pharmaceutical composition of the invention is suitable for industrial production.

The invention is described herein below with reference to accompanying drawings, in which:

FIGS. 1-5 show mass spectrograms of a reaction mixture containing Substance P, butyrylcholinesterase, and Fe⁺³ in water at 0, 25, 60, 120 and 180 minutes;

FIGS. 6-9 show mass spectrograms of a reaction mixture containing Substance P and butyrylcholinesterase in water at 0, 25, 60 and 180 minutes; and

FIGS. 10-15 show mass spectrograms of a reaction mixture containing Substance P and acetylcholinesterase in water at 0, 2, 4, 6, 8 and 30 minutes.

In pharmaceutical compositions of the invention, cholinesterase is preferably selected from acetylcholinesterase; butyrylcholinesterase; acetylcholinesterase or butyrylcholinesterase isozymes, their derivatives; or recombinant cholinesterase enzymes.

In pharmaceutical compositions of the invention, cholinesterase is used as a monomer or a mixture. The sources and purification techniques of cholinesterase are well-known to those skilled in the art, and the sources vary greatly. For example, cholinesterase is isolated from heads of flies, electric eel, bovine serum, animal blood, or from fruit flies by a gene recombination technology (Chinese Patent Nos. 1472316 and 1727478 by reference). Different sources have different cholinesterase content.

Chinese Patent No. 1472316 discloses a method of preparation of acetylcholinesterase for the detection of pesticide residues in fruits and vegetables, comprising collecting enzyme source from animal blood, obtaining crude acetylcholinesterase solution from the enzyme source, purifying acetylcholinesterase with ammonium sulfate, removing ammonium sulfate with dialysis, concentrating and drying the resultant enzyme solution to give a purified acetylcholinesterase.

The term "purified," as used herein, means that an enzyme has been purified to a certain specific activity. Most enzymes may be purified further. Cholinesterase or its isoenzyme as used in embodiments of the invention only needs to be purified to a constant specific activity. Taking acetylcholine or butyrylcholine as substrate, if the enzyme shows activity, it can be used in the embodiments of the invention. In addition, cholinesterase of the invention is commercially available.

The pharmaceutical composition of the invention functions as a tachykinin antagonist, particularly as an antagonist of SP, neurokinin A, neurokinin B or the like. One of the objectives of the invention is to provide a tachykinin antagonist, particularly an SP, neurokinin A or neurokinin B antagonist. The pharmaceutical composition firstly hydrolyzes C-terminal amino acid residues of Substance P from the site of Leu-Met, and then from multiple sites of the C-terminal including tachykinin such as SP, so that tachykinin loses its pharmacologically biological activity or receptor-binding capacity.

After administering the pharmaceutical composition, diseases, such as pain, inflammation, central nervous system diseases (depression, anxiety and schizophrenia), respiratory diseases (asthma), gastrointestinal disorders and gastrointestinal diseases (irritable bowel syndrome and ulcerative colitis), allergy, neuropathological diseases, diseases related to immune enhancement or suppression, rheumatism, skin diseases and chronic fatigue syndrome are cured or prevented, and their symptoms are eliminated.

This invention also relates to a method for the treatment or prevention of tachykinin-mediated diseases in mammals comprising administering to a patient in need thereof a pharmaceutical composition of the invention. The pharmaceutical composition firstly hydrolyzes C-terminal amino acid residues of Substance P from the site of Leu-Met, and then from multiple site of C-terminal of polypeptide including tachykinin such as SP, so that tachykinin loses pharmacologically biological activity or receptor-binding capacity. A tachykinin-mediated disease can be treated or prevented by decreasing tachykinin-mediated immunoregulation or nerve transduction.

The pharmaceutical composition of the invention is a tachykinin antagonist, which is used for the treatment of tachykinin-mediated diseases, including but not limited to pain, inflammation, central nervous system diseases (such as depression, anxiety and schizophrenia), respiratory diseases (such as asthma), gastrointestinal disorders and gastrointestinal diseases (such as irritable bowel syndrome and ulcerative colitis), allergy, neuropathological diseases, diseases related to immune enhancement or suppression, rheumatism, skin diseases, and chronic fatigue syndrome.

Specifically, the pharmaceutical composition of the invention is used for the treatment or prevention of pain, including but not limited to traumatic pain such as postoperative pain; trauma tearing pain in brachial plexus; chronic pain such as arthritis, rheumatoid arthritis, osteoarthritis, or psoriasic arthritis; neuropathic pain such as postherpetic neuralgia, trigeminal neuralgia, segmental or inter-pulse neuralgia, fibromyalgia, burning pain, pain caused by peripheral neuropathy, diabetic neuropathy or chemotherapy-induced neuropathy, occipital neuralgia, geniculate neuralgia, glossopharyngeal neuralgia, reflex sympathetic dystrophy, phantom limb pain; various headaches, such as migraine, acute or chronic tension headache, or cluster headache; toothache; cancer pain; visceral pain; gastrointestinal pain; nerve compression pain; sports injury pain; menstrual pain; pain caused by meningitis and arachnoiditis; musculoskeletal pain; low back pain, such as spinal stenosis, vertebral disc falling, sciatica, pharyngodynia, joint pain caused by ankylosing spondylitis; gout; burns; pain caused by scar or prurigo; cervical pain; shoulder and elbow pain; and thalamic pain such, as thalamic pain caused by stroke.

Specifically, the pharmaceutical composition of the invention is used for the treatment or prevention of inflammation, including but not limited to inflammation caused by asthma, influenza, chronic bronchitis, or rheumatoid arthritis; gastrointestinal inflammation, such as Crohn's disease, ulcerative colitis, colitis, gastritis, or injury induced by non-steroidal anti-inflammatory drugs; skin inflammation, such as caused by herpes or eczema; bladder inflammation, such as cystitis or urinary incontinence; eye or tooth inflammation.

Specifically, the pharmaceutical composition of the invention is used for the treatment or prevention of allergic disorders, including but not limited to allergic skin disorders such as rubella, or allergic respiratory disorders, such as rhinitis.

Specifically, the pharmaceutical composition of the invention is used for the treatment or prevention of schizophrenia, including but not limited to manic, depressive, mixed schizophrenia; paranoid, disorder, tension, mixed and residual schizophrenia; schizophrenia-like illnesses; paranoid mental illness; short-term mental illness; induced psychosis; substance-induced mental illnesses, and unknown mental illnesses.

Specifically, the pharmaceutical composition of the invention is used for the treatment or prevention of severe depression, including but not limited to bipolar depression; unipolar depression; one-time or periodic severe depression with or without mental illness, tension, depression having typical characteristics; and seasonal severe depression. Besides, other emotional disorders belonging to severe depression disorders can be treated in embodiments of the present invention comprising bad mood disorder which occur early or late and with or without typical characteristics, circulation disorder, periodic short-term depressive disorder, mixed affective disorder, neurological depression, posttraumatic stress obstacle or social phobia; vascular obstacle dementia with depression; emotional disorders caused by materials such as alcohol, cocaine, LSD, opium, sedatives, hypnotics, anti-anxiety agents and the like, depression schizophrenia and adjustment disorders with depression.

Specifically, the pharmaceutical composition of the invention is used for the treatment or prevention of anxiety, including but not limited to panic attacks; plaza phobia; specific phobia; social phobia; forced dysfunction; posttraumatic stress disorder; acute stress disorder; general anxiety disorder; anxiety caused by health problems; anxiety caused by materials; and unknown anxiety.

Specifically, the pharmaceutical composition of the invention is used for the treatment or prevention of depression and/or anxiety-related stress disorders, including but not limited to acute stress response; adjustment disorders such as short-term depression, prolonged depression, mixed anxiety and depression, adjustment disorder obviously interfering with other emotions, adjustment disorder having obvious interference, adjustment disorder characterized by mixed interfering emotions and actions and other adjustment disorder having a specific syndrome; and other responses to severe stress.

Specifically, the pharmaceutical composition of the invention is used for the treatment or prevention of sleep disorders, including but not limited to insomnia; sleep apnea; narcolepsy; circadian rhythm disorders; or sleep disorders induced by mental disorders or substance.

Specifically, the pharmaceutical composition of the invention is used for the treatment or prevention of cognitive impairment, including but not limited to dementia; amnesia and other unknown cognitive impairment, particularly dementia caused by degradation disorders, injury, trauma, infection, vascular disease, toxins, anoxia, vitamin deficiency, or endocrine disorders; and amnesia caused by degradation disorders, vascular disease, or the like. Furthermore, the pharmaceutical composition of the invention is used to improve memory and/or cognition for non-cognitive and/or memory-impaired healthy people.

Specifically, the pharmaceutical composition of the invention is used for the treatment or prevention of personality disorders, including but not limited to paranoid personality disorder; schizoid personality disorder; schizophrenic personality disorder; anti-social personality; borderline personality disorder; narcissistic personality disorder; avoidance personality disorder; dependent personality disorder; compulsive personality disorder, and unknown personality disorders.

Specifically, the pharmaceutical composition of the invention is used for the treatment or prevention of eating disorders, including but not limited to anorexia nervosa; atypical anorexia nervosa; bulimia nervosa; atypical bulimia nervosa; excessive eating related to psychological interference; vomiting related to other psychological interference and unknown eating disorders.

Specifically, the pharmaceutical composition of the invention is used for the treatment or prevention of neurodegenerative disease, including but not limited to chronic chorea; demyelinating disorders; other neuropathy and neuralgia; multiple sclerosis; amyotrophic lateral sclerosis; stroke, or head trauma.

Specifically, the pharmaceutical composition of the invention is used for the treatment or prevention of addiction disorders, including but not limited to substance dependence or abuse with or without physiological dependence, particularly when the substance is alcohol, amphetamines, amphetamine-like substances, caffeine, cocaine, hallucinogens, inhalants, nicotine, opium (such as cannabis, heroin and morphine), phencyclidine, or phencyclidine-like substance, sedative, hypnotics, benzodiazepines and/or other substances. Specifically, the composition is used to give up these substances and alcohol, and to stop paranoia.

Specifically, the pharmaceutical composition of the invention is used for the treatment or prevention of emotional disorders, including but not limited to emotional disorders caused by alcohol, amphetamines, caffeine, cannabis, cocaine, hallucinogens, inhalants, nicotine, opium, phencyclidine, sedatives, hypnotics, anti-anxiety drug, or the like.

Specifically, the pharmaceutical composition of the invention is used for the treatment or prevention of sexual dysfunction, including but not limited to sexual desire disorder; erectile dysfunction; sexual orgasm impediment; sexual pain disorder; sexual dysfunction caused by common medical diseases or substances; and sexual dysfunction of unknown origin.

Specifically, the pharmaceutical composition of the invention is used for the treatment or prevention of nausea, retching, or vomiting, including but not limited to acute vomiting, delayed vomiting, anticipatory vomiting; vomiting caused by medicine such as cancer chemotherapy agents; radiation-related vomiting; vomiting caused by radiation therapy, poisons, toxin (such as toxin caused by metabolic disorders or infection such as gastritis, or toxin released during bacterial or viral gastrointestinal infection); pregnancy; vestibular disorder (such as motion sickness, vertigo, dizziness and Meniere's disease); postoperative dizziness; gastrointestinal obstruction; reduced gastric peristalsis; increased intracranial pressure, decreased intracranial pressure (such as mountain sickness); opiate analgesics (such as morphine); gastroesophageal reflux disease; acid indigestion; overindulgence in foods or drinks; acid reflux disease; nausea; heartburn (such as episodic heartburn, nocturnal heartburn and food-induced heartburn); or indigestion.

Specifically, the pharmaceutical composition of the invention is used for the treatment or prevention of skin diseases including but not limited to cutaneous tinea, skin rash, psoriasis, pruritus, insect bite symptoms, and spots, particularly caused by sunburn.

Specifically, the pharmaceutical composition of the invention is used for the treatment or prevention of diseases related to immune enhancement or suppression, including but not limited to rheumatoid arthritis, Crohn's disease, diabetes, lupus, or autoimmune rejection.

Specifically, the pharmaceutical composition of the invention is used for the treatment or prevention of rheumatism, high blood pressure, edema, or chronic fatigue syndrome.

The pharmaceutical composition of the invention is used for the preparation of medicine for the treatment or prevention of schizophrenia, vomiting, anxiety, depression, intestinal stress syndrome, circadian rhythm disorders, pain, neurogenic inflammation, asthma, rheumatic inflammation, inflammation caused by infection, allergy, or pain.

Cholinesterase of the invention can be used as active ingredient of cosmetics for removing freckles, antipruritic and scar preventing agent, and for improvement of skin symptoms.

The diseases mentioned-above are well-known in the literature and have been widely studied.

It should be noted that a therapeutically effective amount of cholinesterase used by professionals affects the treatment or prevention of diseases from which patients suffer. The terms "patient" and "patients," used herein refer, to a mammal, and mammals, respectively, including humans that are suffering from given conditions, diseases, or symptoms.

The term "therapeutically effective amount," used herein, means a certain amount of medicine which can effectively treat, control, or alleviate conditions, diseases, or symptoms. For example, for treating migraine, a therapeutically effective amount should be enough to alleviate migraine or other comfortlessness. A therapeutically effective amount is easily determined by routine experimentation, namely using conventional technology and observing results in similar cycles. When routine experimentation to determine therapeutically effective amount is performed, factors needed considering include but are not limited to species, weight, age, health, disease type, severity, deepness or extent of a disease, individual response, pharmaceutical composition administered, administration mode, biological activity of the pharmaceutical composition, dose, identity of excipients, and so on. For example, when cholinesterase having specific activity of 1,000 U per mg protein is used, the therapeutically effective amount is between 0.01 µg and 1 mg, particularly between 0.1 µg and 1 mg. In general, therapeutically effective amount is determined by skilled artisans.

For the treatment of the above-mentioned diseases, the administration mode of cholinesterase is selected from injection, inhalation, non-intestinal administration, or local administration. For example, cholinesterase can be administered in form of aerosol, spray or dry powder, subcutaneous injection, intramuscular injection, intravenous injection, catheter delivery, nasal administration, rectal administration, vaginal administration, transdermal or mucosal administration, local administration, and the like.

For respiratory diseases such as asthma, a suggested administration mode is in form of aerosol or spray acting on nasal cavity or the respiratory tract. The spray device may be equipped with a heating structure which keeps the solution at 35°C- 45°C so as to improve the activity of enzyme. For central nervous system diseases and chronic fatigue diseases, besides conventional administration mode, cholinesterase of the invention can be administered by oral mucosa in form of aqueous solution, for example, the enzyme solution is contained in the mouth for a while to increase absorption or mixed with xylanase or xylitol as a liquid and sealed in soft capsules. When soft capsules are used, they can be bitten open and the liquid contents allowed remaining in the mouth.

For trauma and surfactant pain, cholinesterase is generally administered directly to pain spot in form of spray, gel, transdermal patch, transdermal absorbent, or by intradermal, subcutaneous or articular cavity injection. For intestinal or reproductive tract disease, cholinesterase may be immersed in an appropriate carrier and slipped into the anus or the reproductive tract. Medical professionals, according to the characteristics of the pharmaceutical composition, the condition of a case, disease development stage, and other relevant circumstances, can easily decide the appropriate dosage form and administration mode. Cholinesterase of the invention can be administered alone or in combination with a pharmaceutically acceptable carrier or exciepient. The proportion and property of each ingredient depend on the solubility, chemical properties of the pharmaceutical composition, administration mode and standard pharmaceutical practice.

In another embodiment of the invention, there provided is a pharmaceutical composition comprising therapeutically effective amount of cholinesterase and one or more pharmaceutically acceptable carrier or excipient. The composition is prepared according to well-known methods disclosed in medical literature. The carrier or excipient is a solid, a semi-solid or a liquid. Appropriate carriers or excipients are disclosed in the literature.

The pharmaceutical composition is suitable for local administration or systemic administration, including oral administration, absorption, non-intestinal administration, or in the form of a capsule, an aerosol, a spray, an inhalant, a suppository, gel, solution agent, suspension, and the like.

For oral administration, the pharmaceutical composition of the invention can be used in combination with a carrier or an excipient and sealed in a capsule. The dosage forms include capsules, suspensions, syrups, mints, chewing gum, or xylitol oral tablet.

The dosage forms at least comprises as active ingredient one ten millionth gram of the enzyme of the invention of which the specific activity is 1,000. Actually, according to demand, the content of active ingredients may account for between 100% and 300% by weight of the unit dosage form. The proportion of the enzyme in a composition is as suitable. The preferred composition and formulation is decided by skilled medical professionals.

For non-intestinal treatment, the pharmaceutical composition may be a solution or suspension. The pharmaceutical composition may be provided as an inhalant, such as aerosol or dry powder which is released in the form of a liquefied or compressed gas or by a pump system. A preferred form of aerosol and dry powder is decided by skilled medical professionals. The pharmaceutical composition of the invention can be administered locally, and excipients such as solution, carbohydrates and liposomes can be incorporated accordingly. Non-intestinal formulation can be provided in an ampoule, dispersed injector, or a glass or plastic multi-dose vial.

For further illustrating the invention, biochemical experiments showing cholinesterase activity, e.g., hydrolysis of SP, and pharmacological experiments showing medical uses of cholinesterase are given below. It should be noted that the following are intended to describe not to limit the invention.

### Example 1

### Hydrolysis of Substance P (SP)

Hydrolysis of SP was performed by acetylcholinesterase and butyrylcholinesterase separately.

Experimental conditions:

1. Cholinesterase

1) Butyrylcholinesterase, specific activity 200 U, 50 mg of dry powder was dissolved in 5 mL of aqueous solution of iron(III) (double distilled water, Fe⁺³ content, 1 mg/L) and centrifuged at 10, 000 r/min for 4 minutes;

2) Butyrylcholinesterase, specific activity 200 U, 50 mg of dry powder was dissolved in 5 mL of double distilled water and centrifuged at 10, 000 r/min for 4 minutes;

3) Acetylcholinesterase, specific activity 300 U, 20 mg of dry powder was dissolved in 1 mL of double distilled water and centrifuged at 10,000 r/min for 4 minutes.

2. Substrate

SP, with purity 98%, in an aqueous solution at a concentration 0.5 µg/mL.

3. Reaction

100 µL of the substrate and 100 µL of enzyme were mixed, and the initial reaction temperature was 22°C.

4. Detection

Flight mass spectrometry: 1 µL of matrix (CHCA) and 1 µL of the reactant were mixed, and the mixture was directly assayed by flight mass spectrometry.

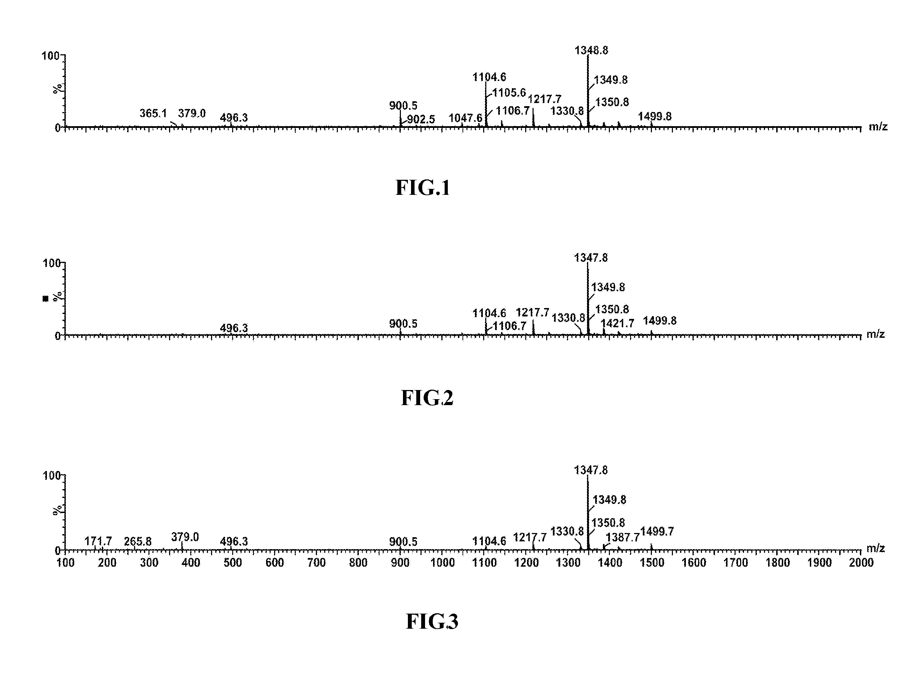

As shown in FIGS. 1-5, a product having a molecular weight of 1217 was detected at 25 minutes, which was the resultant fragment after Met (the molecular weight is 131) was hydrolyzed from SP polypeptide chain (the molecular weight is 1348); new fragments having the molecular weights of 1104 and 900 were detected at 60 minutes, which were the resultant products after Met + Leu (the molecular weight is 113), and Met + Leu + Gly (the molecular weight is 57) + Phe (the molecular weight is 147) were hydrolyzed from SP polypeptide chain.

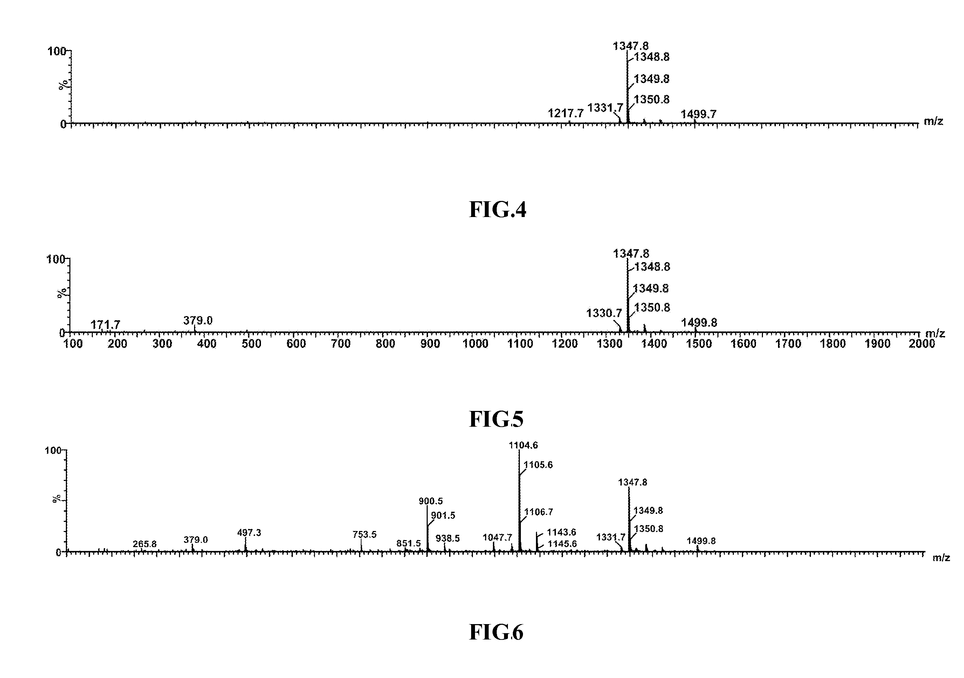

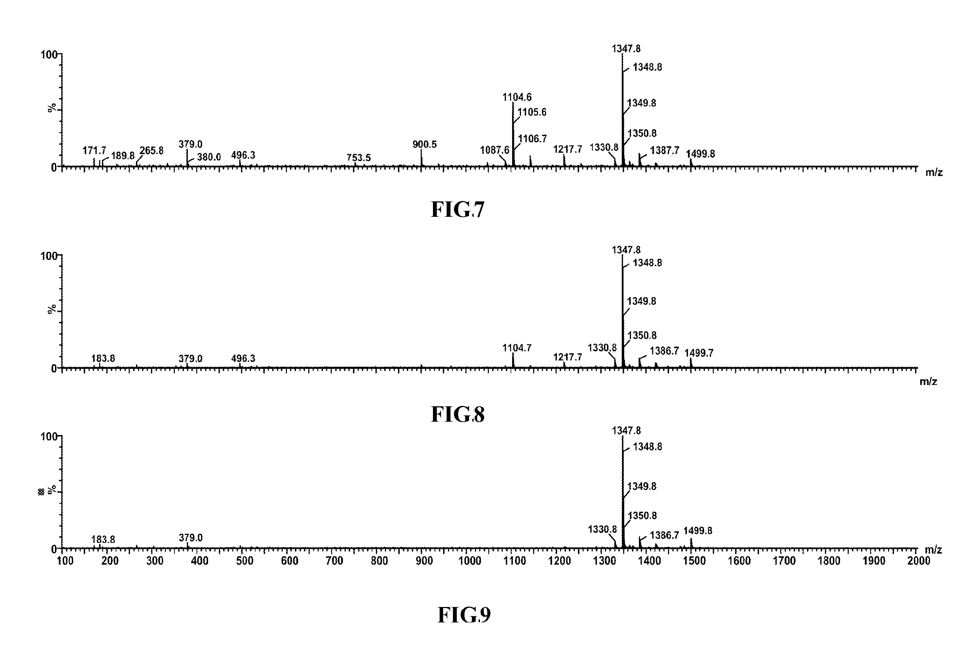

As shown in FIGS. 6-9, products having a molecular weight of 1217 and 1104 were detected at 25 minutes, which were resultant fragments after Met (the molecular weight is 131) and Met + Leu (the molecular weight is 113) were hydrolyzed from the SP polypeptide chain (the molecular weight is 1348). The mass spectrogram in FIG. 3 was similar to that in FIG. 8, except that the former had much more products having a molecular weight of 1104.

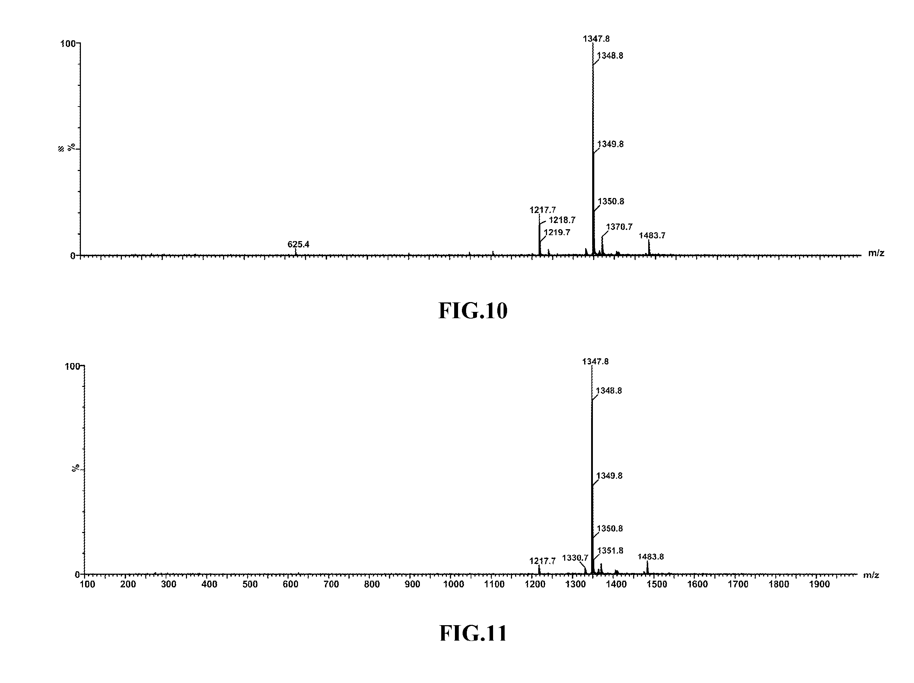

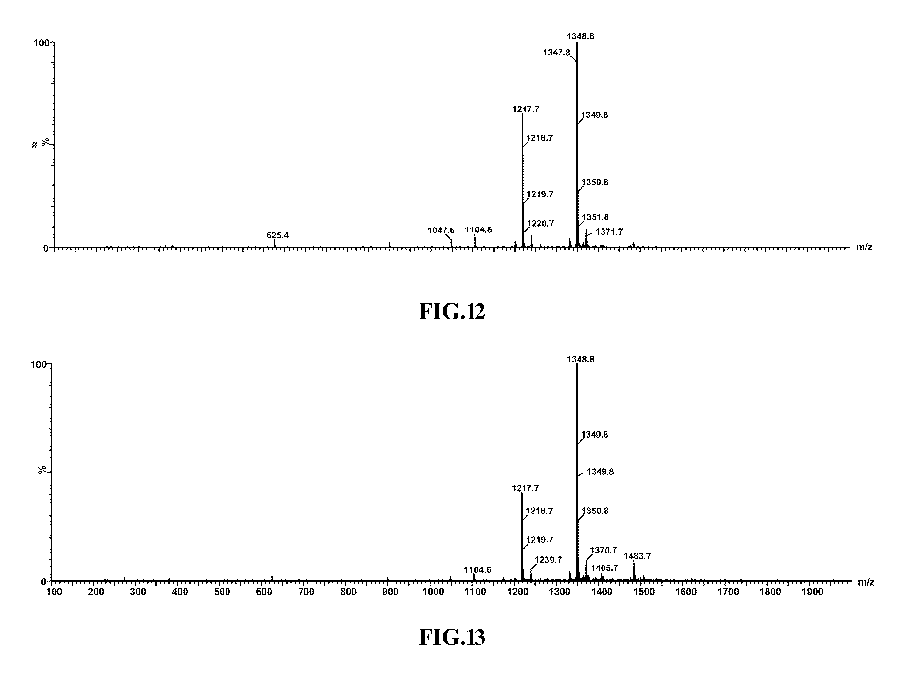

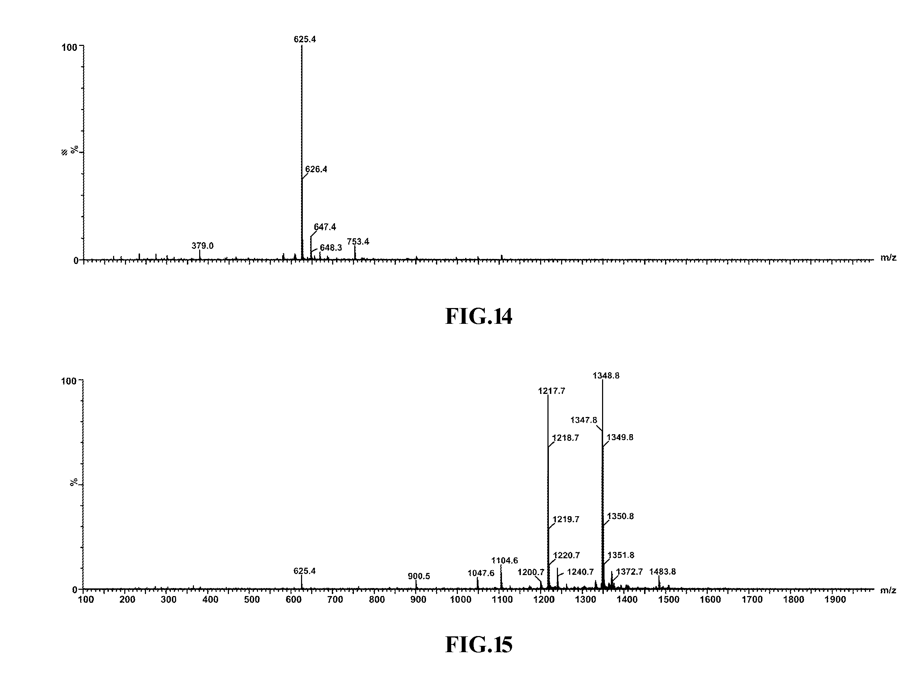

As shown in FIGS. 10-15, in the presence of acetylcholinesterase, C-terminal Met¹¹ of SP was hydrolyzed quickly. The first restriction site was Leu¹⁰-Met¹¹, then Gly⁹-leu¹⁰and Phe⁷-Phe⁸; finally only a 1-5 peptide fragment was left.

From the above experiment it is evident that either acetylcholinesterase or butyrylcholinesterase has the capacity of hydrolyzing SP, and the first restriction site is between Leu¹⁰ and Met¹¹ of SP C-terminal, and the next restriction sites are between Gly⁹-Leu¹⁰, Phe⁷-Phe⁸ and Gln⁵-Gln⁶. It is well-known that if any one of the six C-terminal amino acids is substituted or hydrolyzed, the activity of SP will disappear.

### Example 2

### Preparation of Acetylcholinesterase Aqueous Solution 1

Acetylcholinesterase in the form of freeze-dried powder manufactured by SIGMA was dissolved in water, and propylene glycol functioning as a stabilizer and phenol functioning as an antimicrobial agent were added. The resultant solution was diluted serially by several orders of magnitude until acetylcholinesterase solution having activity between 10 U and 1, 000 U per mg proteins per milliliter of solution was obtained. Depending on mode of administration, the solution was placed in a disposable injector, spraying bottle or other medical bottle, and stored in the cold. The validity period of preservation is up to one year. The enzyme can be used directly or diluted prior to use according to medical requirement.

Specific activity measurement

1. Theoretical basis:

Following the Ellman method, acetylthiocholine iodide (BTCI) was hydrolyzed into thiocholine and acetic acid by cholinesterase (AChE). Thiocholine was reacted with disulfide p-nitrobenzoic acid (DTNB) to yield a yellow product. The resultant product has the largest absorption peak at UV 405 nm, so colorimetric assay can be carried out at this wavelength.

2. Preparation of reagents:

1) phosphate buffer: 0.1 mol/L of disodium hydrogen phosphate and sodium dihydrogen phosphate solution were mixed in an appropriate proportion to yield the pH value of 8.0;

2) cholinesterase solution: based on enzyme activity, the enzyme was dissolved with phosphate buffer, and the A value was adjusted to between 0.4 and 0.8;

3) acetylthiocholine iodide (BTCI) solution: 100 mg of BTCI was dissolved in 10 mL of above-mentioned phosphate buffer (pH 8.0);

4) disulfide p-nitrobenzoic acid (DTNB) solution: 60 mg of DTNB was dissolved in 10 mL of above-mentioned phosphate buffer.

3. Measuring method:

Two test tubes were separately labeled as A and B, and to each tube 3.0 mL of 0.1 mol/L phosphate buffer (pH 8.0), 50 µL of cholinesterase solution, and 50 µL of disulfide p-nitrobenzoic acid (DTNB) were added. The two test tubes were warmed in a water bath at 37°C for 15 min. Then 50 µL of 0.1 mol/L phosphate buffer (pH 8.0) was added to the test tube A as zero standard. 50µL of acetylthiocholine iodide (BTCI) was added to the test tube B, mixed, dumped immediately into a glass cuvette and measured. The OD values before and at 3 minutes were recorded. Each enzyme solution was measured three times, and an average value ΔOD412 was collected for further analysis. Enzyme activity was U= ΔOD412/0.0136*T, wherein T represents reaction time, T=3 (min).

The protein content in the enzyme powder was measured based on Coomassie Brilliant Blue G-250 (GBBG-250) staining method of Bradford .

Specific activity= enzyme activity (U) / mg protein.

### Example 3

### Preparation of Aqueous Solution 2

To aqueous solution 1, 5% propylene glycol and 0.5% laurocapram functioning as penetration agent were added. The solution was administered a transdermal absorption method, such as iontophoresis or with the use of ultrasound.

### Example 4

### Preparation of Glycerol Aqueous Solution

Glycerol aqueous solution was prepared following the method of preparing aqueous solution 1. The proportion of glycerol was between 20% and 70%, and the solution was formulated in different dosage forms, placed in bottles, and stored in the cold or at normal temperature. The maximum usability period at normal temperature was 6 months.

### Example 5

### Preparation of Aqueous Gel

0.8% Polyacrylic acid (carbomer) and 23% glycerol were completely grinded, wetted, and an appropriate amount of water added. Enzyme powder was added according to the proportion of preparing aqueous solution 1, and stirred slowly and uniformly. The resultant product was mainly used for direct applications to skin.

### Example 6

### Analgesic Experiment on Mice by Acetic Acid Twisting Method

1. Materials and reagents

1) Reagents: acetic acid, manufactured by Guangdong Xinning Chemical Plant; butyrylcholinesterase (activity: 200 U per mL solution) prepared following the method of Example 2; Fenbid liniment (ibuprofen), produced by GlaxoSmithKline (China) Investment Co, Ltd., Product batch number: 04050471.

2) Animals: Kunming mice, 40, weight 20 ± 2 g, of either sex.

2. Method

1) Groups:

Saline solution group (negative control group);

Experimental group 1, smeared with butyrylcholinesterase aqueous solution;

Experimental group 2, intraperitoneal injection with butyrylcholinesterase aqueous solution; and

Fenbid group (positive control group).

2) Experimental steps:

40 Kunming mice were randomly grouped with each group 10. The negative control group was administered 0.9% saline solution (0.1 mL/10 g) by intraperitoneal injection. An area of 1×1 cm of the hair of the mice of the experimental group 1 was cut in the back, and butyrylcholinesterase (enzyme activity: 200 U per mL solution) was smeared onto it. The experimental group 2 was administered 0.1 mL/ 10 g butyrylcholinesterase solution (enzyme activity: 200 U per mL solution) by intraperitoneal injection. An area of 1×1 cm of the hair of the mice of the positive control group was cut in the back, and Fenbid was smeared onto it. Each group of mice was fed in different cages. At 5 minutes after administration, following the method taught in Pharmacological Experimental Methodology (3rd Edition) (Xu Shuyun, et al., People's Health Publishing House, 2002, 1), each mouse was administered 0.6% acetic acid (0.1 mg/10 g) by intraperitoneal injection, and the writhing number of mice within 15 minutes was recorded. The number was compared with that of saline solution group (0.9%) to calculate writhing inhibition rate in mice.

3. Results

The results are summarized in Table 1.

**Table 1. Writhing number inhibition on mice by acetic acid twisting method**

| **Groups** | **Writhing number** | **Inhibition rate (%)** |
|---|---|---|
| Saline solution group | 37.1±6.53 | 0 |
| Experimental group 1 | 29.9±5.17* | 24.0 |
| Experimental group 2 | 27.2±9.50*Δ | 26.6 |
| Fenbid group | 35.7±8.40 | |

| | | |
|---|---|---|
| Compared with saline solution group, *P<0.05; compared with Fenbid group, Δ P<0.05. | | |

As shown in Table 1, compared with the saline solution group, butyrylcholinesterase, administered either by intraperitoneal injection or by skin smearing, significantly decreased the writhing number in mice caused by acetic acid intraperitoneal injection (P<0.05). Compared with the positive control group, which was administered Fenbid, an anti-inflammatory and analgesic drug, butyrylcholinesterase aqueous solution administered by intraperitoneal injection also statistically significant (P<0.05), and had a higher inhibition rate on writhing in mice than Fenbid. This shows that butyrylcholinesterase aqueous solution has a good analgesic effect on mice with acute pain.

### Example 7

### Anti-inflammatory Experiment with Adjuvant Arthritis Rats

1. Materials and reagents

1) Reagents: butyrylcholinesterase aqueous solution (activity: 300 U per mL solution) prepared following the method of Example 2; Fenbid liniment (ibuprofen), manufactured by GlaxoSmithKline (China) Investment Co, Ltd., Product batch number: 04050471.

2) Animals: Wister rats, 30, weight 200 ± 2 g, of either sex, from Laboratory Animal Center, Sun Yat-sen University.

2. Method

1) Groups:

Negative control group;

Experimental group; and

Fenbid group.

2) Experimental steps:

Wister rats were randomly grouped into 3 groups with each group of 10. Following the method taught in Pharmacological Experimental Methodology (The third edition) (Xu Shuyun, et al., People's Health Publishing House, 2002, 1), each rat was administered 0.1 mL of Freund's adjuvant (FCA) in the right hindlimb by intradermal injection. After 0.5 h, the experimental group and Fenbid group were separately administered butylcholineterase aqueous solution and Fenbid in the inflammatory site of the right hindlimb, one time per day for a week. At 2 h, 6 h, 12 h, 24 h, 48 h, 72 h and 7 d after administration, the swelling degree of the right hindlimb of rats was separately measured by a vernier caliper.

3. Results

The results are listed in Table 2.

**Table 2 Swelling degree of the right hind limb of rats**

| | **2 h** | **6 h** | **12 h** | **24 h** | **48 h** | **72 h** | **7 d** |
|---|---|---|---|---|---|---|---|
| Negative control group | 0.75 ±0.04 | 0.84 ±0.04 | 0.87 ±0.07 | 0.82 ±0.04 | 0.79 ±0.04 | 0.80 ±0.04 | 0.72 ±0.03 |
| Experimental group | 0.74 ±0.04 | 0.84 ±0.04 | 0.87 ±0.05 | 0.83 ±0.05 | 0.74 ±0.02* | 0.76 ±0.05 | 0.67 ±0.06* |
| Fenbid group | 0.71 ±0.07 | 0.82 ±0.06 | 0.85 ±0.05 | 0.82 ±0.05 | 0.75 ±0.04* | 0.75 ±0.08* | 0.68 ±0.05 |
| Compared with the saline solution group, *P<0.05 | | | | | | | |

As shown in Table 2, compared with the negative control group, at 48 h, 72 h and 7 d after administration, butyrylcholinesterase can significantly decrease the swelling degree of adjuvant arthritis rats (P<0.05), which was similar to the positive control group. This shows that butyrylcholinesterase can reduce inflammatory swelling.

### Example 8

### Prevention and Treatment of Trauma and Inflammation

Acetylcholinesterase (enzyme activity 300 U) was sprayed 1-3 times directly onto a wound. If necessary, gauze pretreated with acetylcholinesterase may also cover the wound. The wound and inflammation in patients was reduced significantly. Pains disappeared. If the wound was not already inflamed, inflammation was not observed, the wound recovered naturally, and a week later dead skin started to fall off. If the wound was inflamed, the inflammation would not develop; three days later, the wound recovered naturally. The mentioned-above wound was a wound caused by trauma, burn or scald.

### Example 9

### Prevention and Treatment of Gingivitis

Patients suffered from swelling gingivitis and pain. Sterile cotton swab was wetted by acetylcholinesterase (enzyme activity 300 U) and placed on the affected area for 10 min, one or two times per day. Two days later, the inflammatory swelling disappeared, without relapse and side effects.

### Example 10

### Prevention and Treatment of Tinea Pedis

To 2 L of warm water lower than 40°C was added 2 mL of acetylcholinesterase aqueous solution (enzyme activity 300 U), and stirred slowly. The feet of a patient were immersed in the solution for 30 minutes, two times per day for three consecutive days. Then the pain disappeared, and one week later, ringworm spot, smell and other symptoms were all gone.

### Example 11

### Prevention and Treatment of Allergic Rhinitis

Acetylcholinesterase (enzyme activity 300 U) glycerol solution was sprayed to the nose of a patient, 3-5 times per day, one spray each time. The course of treatment lasted a week. A month later, in most patients, symptoms such as nasal obstruction, sneezing, runny nose, nose itching and the like disappeared, without relapse. Examination showed inferior turbinate of the patient was not hypertrophic, nasal mucosa was light red, nose clean, without watery secretions.

### Example 12

### Prevention and Treatment of Common Cold

Acetylcholinesterase (enzyme activity 200 U) glycerol solution was sprayed to the nose of a patient who caught a cold at early stage, 3-5 times per day, one spray each time. Within an hour, symptoms such as headache and fever disappeared. Three days later, other symptoms disappeared, and overall wellbeing of the patient recovered to normal.

### Example 13

### Prevention and treatment of summer diarrhea

To 0.5 mL of butyrylcholinesterase (enzyme activity 200 U) aqueous solution, 3 mL of cold water was added. A portion of the solution was placed into the mouth of a patient, and lightly rinsed for 5-10 minutes. Then, the solution was swallowed. Two hours later, the previous steps were repeated. The number of the patients with summer diarrhea decreased greatly, some had no diarrhea at all, and some had only occasional symptoms (1-2 times), then recovered to normal.

This invention is not to be limited to the specific embodiments disclosed herein and modifications for various applications and other embodiments are intended to be included within the scope of the appended claims. While this invention has been described in connection with particular examples thereof, the true scope of the invention should not be so limited since other modifications will become apparent to the skilled practitioner upon a study of the drawings, specification, and following claims.

## Claims

1. A use of cholinesterase for the treatment or prevention of achykinin-mediated diseases in mammals, wherein said diseases comprise pain, inflammation, central nervous system diseases, respiratory diseases, gastrointestinal disorders and gastrointestinal diseases, allergy, neuropathological diseases, diseases related to immune enhancement or suppression, rheumatism, skin diseases and chronic fatigue syndrome.

2. The use of claim 1, wherein said cholinesterase is used as a monomer or a mixture.

3. The use of claim 1, wherein said cholinesterase is selected from the group consisting of acetylcholinesterase, butylcholinesterase, acetylcholinesterase or butylcholinesterase isozymes and derivatives, a recombinant enzyme derivative of acetylcholinesterase or butyrylcholinesterase.

4. The use of claim 1, wherein said cholinesterase is a non-homogeneous crude extract isolated and purified from tissues of animals and plants or blood, or from an expression vector obtained by gene recombinant technology, and has cholinesterase activity when taking acetylcholine or butylcholine as substrate to detect.

5. The use of claim 1, wherein said treatment or prevention comprising hydrolyzing C-terminal amino acid residues of tachykinins comprising Substance P from the site of Leu-Met and multiple sites of C-terminal by cholinesterase, so that tachykinins lose pharmacologically biological activity or receptor-binding capacity and tachykinin-mediated immunoregulation or nerve transduction is decreased.

6. The use of any one of claims 2-4, wherein a therapeutically effective amount of said cholinesterase is between 0.01 µg and 1 mg.

7. A pharmaceutical composition for the treatment or prevention of achykinin-mediated diseases in mammals comprising at least a therapeutically effective amount of cholinesterase of any one of claims 2-4 and a pharmaceutically acceptable carrier.

8. A formulation for antagonizing achykinins in mammals comprising said pharmaceutical composition of claim 7 and a pharmaceutically acceptable excipient.

9. A method of preparation of said formulation of claim 8, comprising homogenously mixing said pharmaceutical composition of claim 7 with a pharmaceutically acceptable excipient.
